# EUROPEAN PATENT SPECIFICATION

(11) **EP 1 478 342 B1**
(45) Date of publication and mention of the grant of the patent: **17.02.2010**
(21) Application number: 02806274.3
(22) Date of filing: 31.12.2002
(51) Int. Cl.: A61K 9/14, A61K 9/12, A61K 31/57, A61K 31/58, A61K 47/34, A61P 11/06

(54) **STERILE FILTERED NANOPARTICULE FORMULATIONS OF BUDESONIDE HAVING TYLOXAPOL AS A SURFACE STABILIZER**
STERILE GEFILTERTE NANOTEILCHEN-FORMULIERUNGEN VON BUDESONID MIT TYLOXAPOL ALS OBERFLÄCHENSTABILISATOR
FORMULATIONS NANOPARTICULAIRES FILTREES STERILES DE BUDESONIDE PRESENTANT DU TYLOXAPOL EN TANT QU'AGENT STABILISATEUR DE SURFACE

(30) Priority: 04.01.2002 US 35324
(43) Date of publication of application: 24.11.2004
(73) Proprietor: Elan Pharma International Limited, Shannon, County Clare (IE)
(72) Inventor: BOSCH, H., William, Bryn Mawr, PA 19010 (US); MARCERA, Donna, M., Limerick, PA 19468 (US); OSTRANDER, Kevin, D., Reading, PA 19606 (US); RYDE, Niels, P., Malvern, PA 19355 (US); WHITE, Douglas, A., King of Prussia, PA 19406 (US)
(74) Representative: Wichmann, Hendrik
(86) International application number: PCT/US2002/041768
(87) International publication number: WO 2003/057194

(56) References cited:
- WO-A-00/27363
- WO-A-97/38699
- US-A- 5 747 001
- OSTRANDER K D ET AL: "An in-vitro assessment of a NanoCrystal ? beclomethasone dipropionate colloidal dispersion via ultrasonic nebulization" EUROPEAN JOURNAL OF PHARMACEUTICS AND BIOPHARMACEUTICS, ELSEVIER SCIENCE PUBLISHERS B.V., AMSTERDAM, NL, vol. 48, no. 3, 1 November 1999 (1999-11-01), pages 207-215, XP004257115 ISSN: 0939-6411
- JACOBS C ET AL: "PRODUCTION AND CHARACTERIZATION OF A BUDESONIDE NANOSUSPENSION FOR PULMONARY ADMINISTRATION" PHARMACEUTICAL RESEARCH, NEW YORK, NY, US, vol. 19, no. 2, February 2002 (2002-02), pages 189-194, XP008015541 ISSN: 0724-8741

## Description

### FIELD OF THE INVENTION

This invention is directed to nanoparticulate compositions of budesonide having tyloxapol as a surface stabilizer, and to methods for the preparation and use of such compositions. The formulations are sterile filtered and are thus useful in pharmaceutical compositions.

### BACKGROUND OF THE INVENTION

### A. Background Regarding Nanoparticulate Compositions

Nanoparticulate compositions, first described in U.S. Patent No. 5,145,684 ("the '684 patent"), are particles consisting of a poorly soluble active agent having adsorbed onto the surface thereof a non-crosslinked surface stabilizer. The '684 patent also describes methods of making such nanoparticulate compositions. Nanoparticulate compositions are desirable because with a decrease in particle size, and a consequent increase in surface area, a composition is rapidly dissolved and absorbed following administration. Methods of making such compositions are described, for example, in U.S. Patent Nos. 5,518,187 and 5,862,999, both for "Method of Grinding Pharmaceutical Substances," U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-Ionic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for "Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,187 for "Method of Grinding Pharmaceutical Substances;" 5,518,738 for "Nanoparticulate NSAID Formulations;" 5,521,218 for "Nanoparticulate Iododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydropropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" and 6,270,806 for "Use of PEG-Derivatized Lipids as Surface Stabilizers for Nanoparticulate Compositions".

Amorphous small particle compositions are described in, for example, U.S. Patent Nos. 4,783,484 for "Particulate Composition and Use Thereof as Antimicrobial Agent," 4,826,689 for "Method for Making Uniformly Sized Particles from Water-Insoluble Organic Compounds," 4,997,454 for "Method for Making Uniformly-Sized Particles From Insoluble Compounds," 5,741,522 for "Ultrasmall, Non-aggregated Porous Particles of Uniform Size for Entrapping Gas Bubbles Within and Methods," and 5,776,496, for "Ultrasmall Porous Particles for Enhancing Ultrasound Back Scatter.

Moreover, WO 00/27363 discloses aqueous dispersions of nanoparticulate aerosol formulations, dry powder nanoparticulate aerosol formulation, propellant-based aerosol formulations, methods of using the formulations in aerosol delivery devices, and methods of making such formulations.

### B. Background Relating to Sterilization of Nanoparticulate Compositions

There are two generally accepted methods for sterilizing pharmaceutical products: heat sterilization and sterile filtration.

### 1. Heat Sterilization of Nanoparticulate Compositions

One of the problems that may be encountered with heat sterilization of nanoparticulate compositions is the solubilization and subsequent recrystallization of the component drug particles. This process results in an increase in the size distribution of the drug particles. In addition, some nanoparticulate formulations also exhibit particle aggregation following exposure to elevated temperatures for heat sterilization.

Crystal growth and particle aggregation in nanoparticulate preparations are highly undesirable for several reasons. The presence of large crystals in the nanoparticulate composition may cause undesirable side effects, especially when the preparation is in an injectable formulation. This is also true for particle aggregation, as injectable formulations preferably have an effective average particle size of no greater than 250 nm. Larger particles formed by particle aggregation and recrystallization can interfere with blood flow, causing pulmonary embolism and death.

In addition, with both injectable and oral formulations the presence of large crystals, and therefore varying particle sizes, and/or particle aggregation can change the pharmacokinetic profile of the administered drug. For oral formulations, the presence of large crystals or aggregates creates a variable bioavailability profile because smaller particles dissolve faster than the larger aggregates or larger crystal particles. A faster rate of dissolution is associated with greater bioavailability and a slower rate of dissolution is associated with a lower bioavailability. This is because bioavailability is proportional to the surface area of an administered drug and, therefore, bioavailability increases with a reduction in the particle size of the dispersed agent (*see* U.S. Patent No. 5,662,833). With a composition having widely varying particle sizes, bioavailability becomes highly variable and inconsistent and dosage determinations become difficult. Moreover, because such crystal growth and particle aggregation are uncontrollable and unpredictable, the quality of the nanoparticulate compositions is inconsistent. For intravenously injected particulate formulations, the presence of large crystals or aggregates can induce an immune systems response which causes the larger particles to be transported by macrophage cells to the liver or spleen and metabolized, in addition to the embolytic effects described above.

Aggregation of nanoparticle compositions upon heating is directly related to the precipitation of the surface stabilizer at temperatures above the cloud point of the surface stabilizer. At this point, the bound surface stabilizer molecules are likely to dissociate from the nanoparticles and precipitate, leaving the nanoparticles unprotected. The unprotected nanoparticles then aggregate into clusters of particles.

Several methods have been suggested in the prior art for preventing such crystal growth and particle aggregation following heat sterilization, including adding a cloud point modifier or crystal growth modifier to the nanoparticulate composition and purifying the surface stabilizer. For example, U.S. Patent No. 5,298,262 describes the use of an anionic or cationic cloud point modifier in nanoparticulate compositions and U.S. Patent No. 5,346,702 describes nanoparticulate compositions having a nonionic surface stabilizer and a non-ionic cloud point modifier. The cloud point modifier enables heat sterilization of the nanoparticulate compositions with low resultant particle aggregation. U.S. Patent No. 5,470,583 describes nanoparticulate compositions having a non-ionic surface stabilizer and a charged phospholipid as a cloud point modifier..

The prior art also describes methods of limiting crystal growth in a nanoparticulate composition by adding a crystal growth modifier (*see* U.S. Patent Nos. 5,662,883 and 5,665,331). In addition, U.S. Patent No. 5,302,401 describes nanoparticulate compositions having polyvinylpyrrolidone (PVP) as a surface stabilizer and sucrose as a cryoprotectant (allowing the nanoparticles to be lyophilized). The compositions exhibit minimal particle aggregation following lyophilization.

All of these various prior art methods share one common feature: they require an additional substance added to the nanoparticulate formulation to inhibit or prevent crystal growth and particle aggregation of the nanoparticulate composition. The addition of such a substance can be detrimental as it may induce adverse effects, particularly for injectable formulations. Thus, this minimizes the usefulness of such substances in pharmaceutical compositions. In addition, the requirement of an additional substance to obtain a stable composition increases production costs.

Another method of limiting particle aggregation or crystal growth of nanoparticulate compositions during sterilization known prior to the present invention was the use of purified surface stabilizers. U.S. Patent No. 5,352,459 describes nanoparticulate compositions having a purified surface stabilizer (having less than 15% impurities) and a cloud point modifier. Purification of surface stabilizers can be expensive and time consuming, thus significantly raising production costs of compositions requiring such stabilizers to produce a stable nanoparticulate composition.

### 2. Sterile Filtration

Filtration is an effective method for sterilizing homogeneous solutions when the membrane filter pore size is less than or equal to about 0.2 microns (200 nm) because a 0.2 micron filter is sufficient to remove essentially all bacteria. Sterile filtration is normally not used to sterilize conventional suspensions of micron-sized drug particles because the drug substance particles are too large to pass through the membrane pores. In principle, 0.2 µm filtration can be used to sterilize nanoparticulate compositions. However, because nanoparticulate compositions have a *size range,* many of the particles of a typical nanoparticulate composition having an average particle size of 200 nm may have a size greater than 200 nm. Such larger particles tend to clog the sterile filter. Thus, only nanoparticulate compositions having very small average particle sizes can be sterile filtered.

### C. Background Relating to Beclomethasone and Budesonide

Budesonide and beclomethasone are anti-inflammatory glucocorticoids useful in the treatment of diseases such as asthma. *See* William E. Serafin, "Therapeutic compounds Used in the Treatment of Asthma", Goodman and Gilman's: The Pharmacological Basis of Therapeutic, Ninth Edition 659-682 (J. G. Hardman et al., eds., McGraw Hill 1996). The prior art discloses the preparation of aerosol formulations of nanoparticulate beclomethasone dipropionate in U.S. Patent No. 5,747,001.

Beclomethasone dipropionate has the following structural formula:

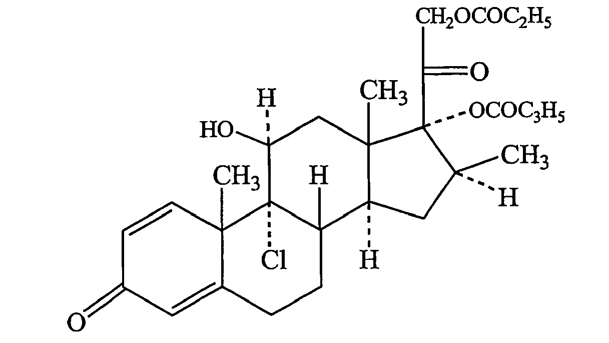

It is a white powder with a molecular weight of 521.25 and is very slightly soluble in water.

Budesonide has the following formula:

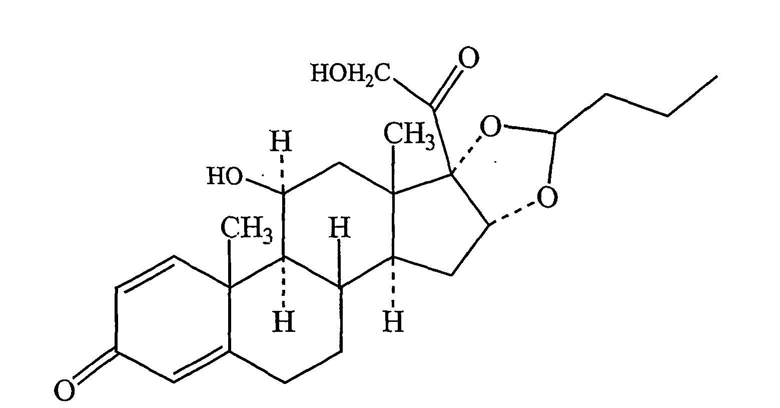

Budesonide is designated chemically as (RS)-11,16, 17,21-Tetrahydroxypregna-1,4-diene-3,20-dione cyclic 16,17-acetal with butraldehyde. Budesonide is provided as the mixture of two epimers (22R and 22S). The empirical formula of budesonide is C₂₅H₃₄O₆ and its molecular weight is 430.5.

Budesonide is a white to off-white odorless powder that is practically insoluble in water and in heptane, sparingly soluble in ethanol, and freely soluble in chloroform.

Glucocorticosteroids have been shown to have a wide range of inhibitory activities against multiple cell types (*e.g*., mast cells, eosinophils, neutrophils, macrophages, and lymphocytes) and mediators (*e.g*., histamine, eicosanoids, leukotrienes and cytokines) involved in allergic and nonallergic/irritant-mediated inflammation. Corticoids affect the delayed (6 hour) response to an allergen challenge more than the histamine-associated immediate response (20 minutes).

### D. Inhalation Treatment with Glucocorticoids

Administration by inhalation of glucocorticoids, compared with oral administration, reduces the risk of systemic side effects. The reduced risk of side effect arises from the mode of administration because glucocorticoids are highly active topically and only weakly active systemically, thereby minimizing effects on the pituitary-adrenal axis, the skin, and the eye. Side effects associated with inhalation therapy are primarily oropharyngeal candidiasis and dysphonia (due to atrophy of laryngeal muscles). Oral glucocorticoids cause atrophy of the dermis with thin skin, striae, and ecchymoses but inhaled glucocorticoids do not cause similar changes in the respiratory tract.

Other advantages of inhaled over oral administration include direct deposition of steroid in the airways which generally provides more predictable administration. The oral doses required for adequate control vary substantially, whereas inhaled glucocorticoids are usually effective within a narrower range. There are, however, a number of factors that influence the availability of inhaled glucocorticoids: extent of airway inflammation; degree of lung metabolism; amount of drug swallowed and metabolized in the GI tract; the patient's ability to coordinate the release and inspiration of the medication; type of glucocorticoid; and the delivery system.

However, the U.S. Food and Drug Administration has recently issued guidelines requiring inhaled products to be sterile. This is problematic for aerosol formulations of nanoparticulate drugs, as heat sterilization can result in crystal growth and particle aggregation, and sterile filtration can be difficult because of the required small particle size of the composition.

There is a need in the art for sterile dosage forms of nanoparticulate budesonide. The present invention satisfies this need.

### SUMMARY OF THE INVENTION

The present invention is directed to the unexpected discovery that nanoparticulate compositions of budesonide having tyloxapol as a surface stabilizer can be readily sterilized by sterile filtration. The invention relates to compositions and methods as defined in the claims.

The compositions of the invention comprise nanoparticulate budesonide having tyloxapol as a surface stabilizer. The compositions may also include one or more secondary surface stabilizers adsorbed onto the surface of the drugs.

The nanoparticulate compositions have an optimal effective average particle size of less than 150 nm, less than 120 nm, less than 100 nm, less than 80 nm, or less than 50 nm. Because the compositions have such a small effective average particle size, they can be readily sterile filtered.

Another aspect of the present invention is directed to a method of making the nanoparticulate compositions of the invention. Such a method comprises contacting budesonide with tyloxapol, and if desired one or more secondary surface stabilizers, for a time and under conditions sufficient to obtain a nanoparticulate composition having the desired particle size. The compositions can then be sterile filtered.

Yet another aspect of the invention is directed to a pharmaceutical composition comprising a sterile filtered nanoparticulate composition of the invention. The pharmaceutical composition comprises a therapeutically effective amount of a nanoparticulate composition of the invention in admixture with a pharmaceutically acceptable carrier.

Still another aspect of the present invention is directed to a nanoparticulate composition for use in a method of treating a mammal suffering from a condition for which budesonide is indicated, comprising administering to the mammal a therapeutically effective amount of a pharmaceutical composition of the present invention.

Both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPTION OF THE PREFERRED EMBODIMENTS

The present invention is directed to nanoparticulate compositions of budesonide having tyloxapol as a surface stabilizer, and optionally one or more secondary surface stabilizers. Surprisingly, the compositions have extremely small effective average particle sizes, which allow the compositions to be sterile filtered.

As taught in the '684 patent, not every combination of surface stabilizer and drug will result in a stable nanoparticulate composition. The discovery of the present invention is surprising as other surface stabilizers were found to be ineffective in attempts to make nanoparticulate compositions of beclomethasone and budesonide. Such stabilizers include hydroxypropyl methylcellulose, methyl cellulose, Pluronic F108^{®}, polysorbates 20 and 80, and polyvinylpyrrolidine.

Even more surprising is that even when a nanoparticulate composition of budesonide and beclomethasone having one or more of the non-tyloxapol surface stabilizers was made, such a nanoparticulate composition could not successfully be sterile filtered.

Finally, it was surprisingly discovered that not all steroids having tyloxapol as a surface stabilizer can be reduced to a particle size small enough to be sterile filtered, as demonstrated by experiments with flunisolide and triamcinolone acetonide. Thus, the discovery of the present invention does not extend to a class of compounds; but rather is limited to the steroid budesonide.

### A. Nanoparticulate Compositions

The compositions of the invention comprise budesonide as active agents, having tyloxapol adsorbed on the surface of the active agents as a surface stabilizer. One or more secondary surface stabilizers may also be adsorbed thereon. Such surface stabilizers physically adhere to the surface of the nanoparticulate active agent, but do not chemically react with the active agent or with each other. Individually adsorbed molecules of the surface stabilizer are essentially free of intermolecular crosslinkages.

As used herein, the term beclomethasone means free beclomethasone and its various mono- and diesters. Specifically included is beclomethazone dipropionate and its monohydrate. The term budesonide means free budesonide and its various mono- and diesters.

Budesonide may be given in a high inhaled dose with very low systemic effects, possibly because of its rapid metabolism. The high rapid systemic elimination of budesonide is due to extensive and rapid hepatic metabolism. Long term clinical studies have shown that inhaled budesonide is a pharmacologically safe drug. High doses of inhaled budesonide are highly effective and well tolerated when used in oral steroid replacement therapy. In addition, budesonide has exhibited benefits of long term control of asthma.

Beclomethasone and budesonide have a high affinity for intracellular glucocorticoid receptors but are rapidly metabolized to biologically inactive compounds. Asthma can usually be controlled with daily inhaled doses of beclomethasone or budesonide in the range of 200 to 800 micrograms. Doses up to 1000 microgram daily have little effect on pituitary-adrenal secretion in adults; larger doses may cause some (variable) dose-dependent suppression of secretion. Doses of 2000 microgram/day in adults have been associated with thinning of the skin, slight glucose intolerance, psychiatric disturbances (rarely), and cataracts (with long-term therapy). Beclomethasone in doses of 1000 to 2000 microgram/day (long term) has been associated with decreases in bone density.

The present invention also includes the nanoparticulate compositions of the invention formulated into pharmaceutical compositions together with one or more non-toxic physiologically acceptable carriers, adjuvants, or vehicles, collectively referred to as carriers, for parenteral injection, for oral administration in solid or liquid form, for rectal or topical administration, inhalable or nasal aerosol administration, and the like.

### 1. Surface Stabilizers

The nanoparticulate budesonide has tyloxapol as a surface stabilizer adsorbed onto the surface of the drug particles. Tyloxapol is a (4-(1,1,3,3,-tetramethylbutyl)-phenol polymer with ethylene oxide and formaldehyde, which is a nonionic liquid polymer of the alkyl aryl polyether alcohol type, and is also known as superinone or triton. Tyloxapol is commercially available and/or can be prepared by techniques known in the art.

Tyloxapol is disclosed as being a useful nonionic surface active agent in a lung surfactant composition in U.S. Patent No. 4,826,821 and as a stabilizing agent for 2-dimethylaminoethyl 4-n-butylaminobenzoate in U.S. Patent No. 3,272,700. In addition, tyloxapol is taught as being a useful surface stabilizer for nanoparticulate compositions in U.S. Patent No. 5,429,824.

In addition to tyloxapol as a surface stabilizer, optional secondary surface stabilizers are also contemplated. Useful secondary surface stabilizers include various polymers, low molecular weight oligomers, natural products, and surfactants. Preferred surface stabilizers include nonionic and ionic surfactants. Two or more secondary surface stabilizers may be employed in combination.

Representative examples of secondary surface stabilizers include cetyl pyridinium chloride, gelatin, casein, lecithin (phosphatides), dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e.g.*, the commercially available Tweens® such as Tween 20® and Tween 80® (ICI Specialty Chemicals)); polyethylene glycols (*e.g*., Carbowaxs 3350® and 1450®, and Carbopol 934® (Union Carbide)), dodecyl trimethyl ammonium bromide, polyoxyethylene stearates, colloidal silicon dioxide, phosphates, sodium dodecylsulfate, carboxymethylcellulose calcium, hydroxypropyl celluloses (*e.g.*, HPC, HPC-SL, and HPC-L), hydroxypropyl methylcellulose (HPMC), carboxymethylcellulose sodium, methylcellulose, hydroxyethylcellulose, hydroxypropylcellulose, hydroxypropylmethyl-cellulose phthalate, noncrystalline cellulose, magnesium aluminum silicate, triethanolamine, polyvinyl alcohol (PVA), polyvinylpyrrolidone (PVP), poloxamers (*e.g*., Pluronics F68® and F108®, which are block copolymers of ethylene oxide and propylene oxide); poloxamines (*e.g*., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); a charged phospholipid such as dimyristoyl phophatidyl glycerol; dioctylsulfosuccinate (DOSS); Tetronic 1508® (T-1508) (BASF Wyandotte Corporation); dialkylesters of sodium sulfosuccinic acid (*e.g.*, Aerosol OT®, which is a dioctyl ester of sodium sulfosuccinic acid (American Cyanamid)); Duponol P®, which is a sodium lauryl sulfate (DuPont); Tritons X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-10G® or Surfactant 10-G® (Olin Chemicals, Stamford, CT); Crodestas SL-40® (Croda, Inc.); SA9OHCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; random copolymers of vinyl acetate and vinyl pyrrolidone, such as Plasdone® S630, and the like.

Particularly preferred secondary surface stabilizers are DOSS, sodium lauryl sulfate, hydroxypropylmethyl cellulose, benzalkonium chloride, and polyvinylpyrrolidine.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 1990).

The surface stabilizers are commercially available and/or can be prepared by techniques known in the art.

### 2. Nanoparticulate Budesonide/Surface Stabilizer Particle Size

The nanoparticulate compositions of the invention comprise nanoparticulate budesonide having an effective average particle less than 150 nm, less than 120 nm, less than 100 nm, less than 80 nm, or less than 50 nm, as measured by conventional particle size measuring techniques well known to those skilled in the art. Such techniques include, for example, sedimentation field flow fractionation, photon correlation spectroscopy, light scattering, and disk centrifugation.

By "an effective average particle size less than 150 nm" it is meant that at least 50% of the active agent particles have a weight average particle size of less than 150 nm when measured by the above techniques. Preferably, at least 70%, 90%, or 95% of the active agent particles have an average particle size of less than 150 mn.

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents are lactose monohydrate, lactose anhydrous, and various starches. Examples of binding agents are various celluloses and cross-linked polyvinylpyrrolidone, microcrystalline cellulose, such as Avicel^{®} PH101 and Avicel^{®} PH102, microcrystalline cellulose, and silicifized microcrystalline cellulose (SMCC).

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

### 4. Concentration of Nanoparticulate Budesonide and Tyloxapol

The relative amount of budesonide and tyloxapol can vary widely. The optimal amount of drug and tyloxapol can depend, for example, upon the presence of secondary surface stabilizers, the particular intended dosage form, etc.

The concentration of tyloxapol can vary from 0.01 to 90%, from 1 to 75%, from 10 to 60%, or from 10 to 30% by weight, based on the total combined dry weight of the budesonide and tyloxapol.

The concentration of the budesonide can vary from about 99% to 1%, from 90% to 10%, from 80% to 30%, or from 80% to 40% by weight, based on the total combined dry weight of the budesonide and tyloxapol.

### B. Methods of Making Nanoparticulate Formulations

The nanoparticulate budesonide compositions of the invention can be made using, for example, milling, precipitation, or microfluidization techniques. Exemplary methods of making nanoparticulate compositions are described in the '684 patent. Methods of making nanoparticulate compositions are also described in U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" U.S. Patent No. 5,665,331, for "Co-Microprecipitation of Nanoparticulate Pharmaceutical Agents with Crystal Growth Modifiers;" U.S. Patent No. 5,662,883, for "Co-Microprecipitation of Nanoparticulate Pharmaceutical Agents with Crystal Growth Modifiers;" U.S. Patent No. 5,560,932, for "Microprecipitation of Nanoparticulate Pharmaceutical Agents;" U.S. Patent No. 5,543,133, for "Process of Preparing X-Ray Contrast Compositions Containing Nanoparticles;" U.S. Patent No. 5,534,270, for "Method of Preparing Stable Drug Nanoparticles;" U.S. Patent No. 5,510,118, for "Process of Preparing Therapeutic Compositions Containing Nanoparticles;" and U.S. Patent No. 5,470,583, for "Method of Preparing Nanoparticle Compositions Containing Charged Phospholipids to Reduce Aggregation".

### 1. Milling to obtain Nanoparticulate Drug Dispersions

Milling of aqueous budesonide to obtain a nanoparticulate dispersion comprises dispersing budesonide particles in a liquid dispersion medium, followed by applying mechanical means in the presence of grinding media to reduce the particle size of the active agents to the desired effective average particle size.

The liquid dispersion medium can be any medium in which the active agent particles are poorly soluble. By "poorly soluble" it is meant that the drug has a solubility in the liquid dispersion medium of less than about 10 mg/ml, and preferably of less than about 1 mg/ml. A preferred liquid dispersion medium is water. However, the invention can also be practiced with other liquid media in which the drug is poorly soluble and dispersible including, for example, aqueous salt solutions, safflower oil, and solvents, such as ethanol, t-butanol, hexane, and glycol.

The active agent particles can be reduced in size in the presence of tyloxapol and optionally one or more secondary surface stabilizers. Alternatively, the active agent particles can be contacted with tyloxapol and optionally one or more secondary surface stabilizers after attrition. Other compounds, such as a diluent, can be added to the active agent/surface stabilizer composition during the size reduction process. Dispersions can be manufactured continuously or in a batch mode.

### 2. Precipitation to Obtain Nanoparticulate Drug Compositions

Another method of forming the desired nanoparticulate composition is by microprecipitation. This is a method of preparing stable dispersions of budesonide or belcomethasone in the presence of tyloxapol, optionally one or more secondary surface stabilizers, and one or more colloid stability enhancing surface active agents free of any trace toxic solvents or solubilized heavy metal impurities. Such a method comprises, for example: (1) dissolving the active agent in a suitable solvent; (2) adding the formulation from step (1) to a solution comprising tyloxapol and optionally one or more secondary surface stabilizers to form a clear solution; and (3) precipitating the formulation from step (2) using an appropriate non-solvent. The method can be followed by removal of any formed salt, if present, by dialysis or diafiltration and concentration of the dispersion by conventional means. The resultant nanoparticulate active agent dispersion can be utilized in solid or liquid dosage formulations.

### 3. Microfluidization to Obtain Nanoparticulate Drug Compositions

U.S. Patent No. 5,510,118, for "Process of Preparing Therapeutic Compositions Containing Nanoparticles," describes an exemplary method of making nanoparticulate compositions using microfluidization techniques.

### 4. Sterile Filtration

The nanoparticulate active agent composition can be sterile filtered using conventional means. Sterile filters have pore sizes of about 0.2 microns or less, which is small enough to filter out biological contaminants. Suitable filters are commercially available.

Following sterile filtration, the nanoparticulate composition can be utilized in solid or liquid dosage formulations, such as controlled release dosage formulations, solid dose fast melt formulations, aerosol formulations, tablets, capsules, etc.

The compositions are particularly useful for dosage forms in which sterility is of primary importance, such as liquid aerosols and injectable formulations.

### C. Methods of Using the Nanoparticulate Compositions

The nanoparticulate compositions of the present invention can be administered to humans and animals either orally, rectally, parenterally (intravenous, intramuscular, or subcutaneous), intracisternally, intravaginally, intraperitoneally, locally (powders, ointments or drops), or as a buccal, inhalable, or nasal spray. The budesonide nanoparticulate compositions may be used in the treatment of mammals suffering from inflammatory diseases. Nanoparticulate compositions of this invention administered as inhalable aerosols are also contemplated and can be particularly useful in the treatment of respiratory illnesses, such as asthma, cystic fibrosis, chronic obstructive pulmonary disease (COPD), etc.

Pharmaceutical compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles include water, ethanol, polyols (propyleneglycol, polyethyleneglycol, glycerol, and the like), vegetable oils (such as olive oil), injectable organic esters such as ethyl oleate, and suitable mixtures thereof.

The nanoparticulate compositions may also contain adjuvants such as preserving, wetting, emulsifying, and dispensing agents. Prevention of the growth of microorganisms can be ensured by various antibacterial and antifungal agents, such as parabens, chlorobutanol, phenol, sorbic acid, and the like. It may also be desirable to include isotonic agents, such as sugars, sodium chloride, and the like. Prolonged absorption of the injectable pharmaceutical form can be achieved by the use of agents delaying absorption, such as aluminum monostearate and gelatin.

Exemplary solid dosage forms for oral administration include capsules, tablets, pills, powders, and granules.

Liquid dosage forms for oral administration include pharmaceutically acceptable emulsions, solutions, suspensions, syrups, and elixirs. In addition to the nanoparticulate compositions, the liquid dosage forms may comprise inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, corn germ oil, olive oil, castor oil, and sesame oil, glycerol, tetrahydrofurfuryl alcohol, polyethyleneglycols, fatty acid esters of sorbitan, or mixtures of these substances, and the like.

Actual dosage levels of active ingredients in the nanoparticulate compositions of the invention may be varied to obtain an amount of active ingredient that is effective to obtain a desired therapeutic response for a particular composition and method of administration. The selected dosage level therefore depends upon the desired therapeutic effect, the route of administration, the potency of the administered therapeutic compound, the desired duration of treatment, and other factors. Dosage unit compositions may contain such amounts of such submultiples thereof as may be used to make up the daily dose.

The following examples are given to illustrate the present invention. It should be understood, however, that the invention is not to be limited to the specific conditions or details described in these examples.

### Example 1

The purpose of this example was to prepare a sterile filtered nanoparticulate budesonide composition stabilized with tyloxapol.

Budesonide (25 g) was dispersed in an aqueous solution of tyloxapol (4.97 g) in deionized water (469.9 g). The pH of the slurry was adjusted to 4.1 with 1 M acetic acid. The slurry was process in a DYNO®-Mill (Willy A. Bachofen AG) assembled with a 300 cc chamber for continuous milling and charged with 500 µm Sdy-20 polymeric milling media (Eastman Kodak). The chamber and process fluid vessel were cooled with 10°C coolant. Milling was performed at 4200 rpm.

After 8 hours the dispersion had a mean particle size of 161 nm and was harvested. Approximately 95 g of this dispersion was then combined with 130 cc of 50 µm SDy20 polymeric media, charged into the 150 cc batch chamber of a DYNO®-Mill, and milled at 4200 rpm. After 2 hours of milling the material was harvested and had a mean particle size of 80 nm.

The nanoparticulate budesonide dispersion was filtered through several Gelman Acrodisc PF 0.8/0.2 µm syringe filters. The mean particle size of the filtered dispersion was 83 nm, indicating that the filtration process did not significantly change the particle size distribution of the nanoparticulate budesonide dispersion.

### Example 2

The purpose of this example was to prepare a sterile filtered nanoparticulate budesonide composition stabilized with tyloxapol and the secondary surface stabilizer hydroxypropylmethylcellulose (HPMC).

Budesonide (8.5 g) was dispersed in an aqueous solution of tyloxapol (0.85 g) and HPMC (Pharmacoat® 603; Shin-Etsu) in deionized water (74.8 g). The slurry was combined with 130 mL of 500 µm SDy20 polymeric media and charged into the 150 cc batch chamber of a DYNO®-Mill. Milling was performed at 4200 rpm. After 185 minutes the dispersion was harvested and had a mean particle size of 137 nm.

Approximately 42.5 g of this nanoparticulate budesonide dispersion was diluted with 42.5 g of deionized water and then combined with 130 mL of 50 µm SDy20 polymeric media. The material was charged into the 150 cc batch chamber of a DYNO®-Mill and milled at 4200 rpm. After 80 minutes of milling the nanoparticulate budesonide dispersion had a mean particle size of 90 nm and was harvested.

A portion of the harvested nanoparticulate budesonide dispersion was filtered through a 0.2 µm syringe filter. The mean particle size of the filtered dispersion was 87 nm, indicating that the filtration process did not significantly change the particle size distribution of the nanoparticulate budesonide dispersion.

### Example 3

The purpose of this example was to prepare a sterile filtered nanoparticulate budesonide composition stabilized with tyloxapol using a high speed disperser.

Budesonide (210 g) was dispersed in an aqueous solution of tyloxapol (21 g) in Sterile Water for Injection, USP (819 g), and the slurry was then charged into the vessel of a Hockmeyer 5 L High Speed Disperser (Hockmeyer Equip. Corp., Harrison, NJ). The system was placed under vacuum (20-25" Hg) and then charged with 1365 g of 50 µm SDy20 polymeric media. Milling was performed at 7000 rpm using a centered 3" Valynn blade. After 27 hours of milling the nanoparticulate budesonide dispersion had a mean particle size of 80 nm.

The nanoparticulate budesonide dispersion was diluted to a nominal budesonide concentration of 5% w/w and discharged. Benzalkonium chloride and acetic acid were added to the dispersion at concentrations of 0.01% w/w and 0.02%, respectively. The harvested nanoparticulate budesonide dispersion was filtered through a Gelman SuporCap 0.8/0.2 µm sterilizing grade capsule filter and assayed for budesonide concentration which was found to be 5.0% w/w.

### Example 4

The purpose of this example was to prepare a sterile filtered nanoparticulate budesonide composition stabilized with tyloxapol and the secondary surface stabilizer polyvinylpyrrolidone using a high speed disperser.

Budesonide (210 g) was dispersed in an aqueous solution of tyloxapol (21 g) and polyvinylpyrrolidone (21 g) in Sterile Water for Injection, USP (798 g), and the slurry was then charged into the vessel of a Hockmeyer 5 L High Speed Disperser. The system was placed under vacuum (20-25" Hg) and then charged with 1365 g of 50 µm SDy20 polymeric media. Milling was performed at 7000 rpm using a centered 3" Valynn blade. After 27 hours of milling the nanoparticulate budesonide dispersion had a mean particle size of 80 nm.

The nanoparticulate budesonide dispersion was diluted to a nominal budesonide concentration of 5% w/w and discharged. Benzalkonium chloride and acetic acid were added to the dispersion at concentrations of 0.01% w/w and 0.02%, respectively. The harvested nanoparticulate budesonide dispersion was filtered through a Gelman SuporCap 0.8/0.2 µm sterilizing grade capsule filter and assayed for budesonide concentration which was found to be 5.0% w/w.

### Example 5 (reference example)

The purpose of this example was to demonstrate the inability to sterile filter a nanoparticulate composition of budesonide stabilized with hydroxypropyl methylcellulose.

Budesonide (8.54 g) was dispersed in an aqueous solution of hydroxypropylmethyl cellulose (Methocel E3 Premium LV; Dow Chemical) (1.72 g) in deionized water (74.83 g). Approximately 75 g of the slurry was combined with 130 cc of 500 µm SDy20 polymeric media and charged into the 150 cc batch chamber of a DYNO®-Mill. Milling was performed at 4200 rpm After 4 hours the dispersion had a mean particle size of 128 nm.

The nanoparticulate budesonide dispersion was harvested and diluted with water to yield 79 g of a dispersion with a nominal budesonide concentration of 5% w/w.

Approximately 75 g of this nanoparticulate budesonide dispersion was then combined with 140 mL of 50 µm SDy20 polymeric media. An additional 10 mL of deionized water was added to reduce the viscosity of the dispersion. The material was charged into the 150 cc batch chamber of a DYNO®-Mill and milled at 4200 rpm. After 4 hours of milling the material was harvested and diluted with an additional 36 mL of water. The resulting nanoparticulate budesonide dispersion had a mean particle size of 89 nm but was somewhat aggregated.

An attempt was made to filter a small aliquot through a 25 mm Gelman Supor 0.8/0.2 polyethersulfone syringe filter, however the effluent was clear indicating that the therapeutic compound particles were unable to pass through the filter pores.

### Example 6 (reference example)

The purpose of this example was to demonstrate the inability to sterile filter a nanoparticulate budesonide composition stabilized with methyl cellulose.

Budesonide (8.5 g) was dispersed in an aqueous solution of methyl cellulose (Methocel A15 Premium LV; Dow Chemical) (1.72 g) in deionized water (74.94 g). The slurry was combined with 130 mL of 500 µm SDy20 polymeric media and charged into the 150 cc batch chamber of a DYNO®-Mill. Milling was performed at 4200 rpm. After 4 hours the nanoparticulate budesonide dispersion was harvested and diluted with ca. 30 mL of water to yield a nominal budesonide concentration of 5% w/w.

The nanoparticulate budesonide dispersion had a mean particle size of 170 nm. Approximately 60 g of this dispersion was then combined with 120 mL of 50 µm SDy20 polymeric media. An additional 10 mL of deionized water was added to increase the volume of the dispersion. The material was charged into the 150 cc batch chamber of a DYNO®-Mill and milled at 4200 rpm. After 2 hours of milling the material was harvested and diluted with an additional 40 mL of water to reduce the viscosity.

The resulting nanoparticulate budesonide dispersion consisted of aggregated budesonide particles having an average size of approximately 2 microns, and was therefore unsuitable for 0.2 µm sterile filtration.

### Example 7 (reference example)

The purpose of this example was to demonstrate the inability to sterile filter a nanoparticulate budesonide composition stabilized with Pluronic® F 108.

Budesonide (8.51 g) was dispersed in an aqueous solution of Pluronic® F108 (1.69 g) in deionized water (74.84 g). The slurry was combined with 130 cc of 500 µm SDy20 polymeric media and charged into the 150 cc batch chamber of a DYNO®-Mill. Milling was performed at 4200 rpm. After 4 hours the nanoparticulate budesonide dispersion had a mean particle size of 276 nm.

The nanoparticulate budesonide dispersion was immediately harvested and found to have a mean particle size of 739 nm, indicating that very rapid crystal growth had occurred. Due to this instability, the nanoparticulate budesonide dispersion was deemed unsuitable for 0.2 µm sterile filtration.

### Example 8 (reference example)

The purpose of this example was to demonstrate the inability to sterile filter a nanoparticulate budesonide composition stabilized with polysorbate 80.

Budesonide (8.5 g) was dispersed in a solution of polysorbate 80 (1.7 g) in aqueous diluent (74.8 g). The slurry was combined with 130 mL of 500 µm SDy20 polymeric media and charged into the 150 cc batch chamber of a DYNO®-Mill. Milling was performed at 4200 rpm. After 2 hours the nanoparticulate budesonide dispersion was harvested and had a mean particle size of 221 nm.

Approximately 42.5 g of this dispersion was diluted with 42.5 g of aqueous diluent and then combined with 120 mL of 50 µm SDy20 polymeric media. The material was charged into the 150 cc batch chamber of a DYNO®-Mill and milled at 4200 rpm. After 1 hour of milling the nanoparticulate budesonide dispersion had a mean particle size of 216 nm, and after 2 hours the average size had decreased to only 192 nm, indicating that no significant additional particle size reduction had taken place.

Because of the large average particle size of the nanoparticulate budesonide dispersion, the material was unsuitable for 0.2 µm sterile filtration.

### Example 9 (reference example)

The purpose of this example was to demonstrate the inability to sterile filter a nanoparticulate budesonide composition stabilized with polysorbate 80 and polyvinylpyrrolidone.

Budesonide (8.5 g) was dispersed in an aqueous solution of polysorbate 80 (0.85 g) and polyvinylpyrrolidone (0.85 g) in deionized water (74.8 g). The slurry was combined with 130 mL of 500 µm SDy20 polymeric media and charged into the 150 cc batch chamber of a DYNO®-Mill. Milling was performed at 4200 rpm. After 180 minutes the nanoparticulate budesonide dispersion was harvested and had a mean particle size of 232 nm.

Approximately 40 g of this nanoparticulate budesonide dispersion was diluted with 40 g of deionized water and then combined with 120 mL of 50 µm SDy20 polymeric media. The material was charged into the 150 cc batch chamber of a DYNO®-Mill and milled at 4200 rpm. After 180 minutes of milling the nanoparticulate budesonide dispersion was harvested and had a mean particle size of 203 nm.

Because of the large average particle size of the nanoparticulate budesonide dispersion, the material was unsuitable for 0.2 µm sterile filtration.

### Example 12 (reference example)

The purpose of this example was to demonstrate the inability to sterile filter a nanoparticulate beclomethasone composition stabilized with polysorbate 80.

Beclomethasone dipropionate (4.50 g) was dispersed in an aqueous solution of polysorbate 20 (0.90 g) in deionized water (84.6 g). The slurry was combined with 130 mL of 500 µm SDy20 polymeric media and charged into the 150 cc batch chamber of a DYNO®-Mill. Milling was performed at 4200 rpm. After 125 minutes the dispersion had a mean particle size of 241 nm.

The nanoparticulate beclomethasone dispersion was immediately harvested and the mean particle size was found to have increased to 375 nm, indicating that very rapid crystal growth had occurred. Due to this instability, the nanoparticulate beclomethasone dispersion was deemed unsuitable for 0.2 µm sterile filtration.

### Example 13 (reference example)

The purpose of this example was to demonstrate the inability to sterile filter a nanoparticulate beclomethasone composition stabilized with polysorbate 20.

Beclomethasone dipropionate (4.50 g) was dispersed in an aqueous solution of polysorbate 20 (0.90 g) in deionized water (84.61 g). The slurry was combined with 130 mL of 500 µm SDy20 polymeric media and charged into the 150 cc batch chamber of a DYNO®-Mill. Milling was performed at 4200 rpm. After 1 hour the dispersion had a mean particle size of 212 nm, and after 2 hours the average size had decreased to only 193 nm indicating that no significant additional particle size reduction had taken place. Furthermore, the dispersion was significantly aggregated.

Because of the large average particle size of the nanoparticulate beclomethasone dispersion and its degree of aggregation, the material was unsuitable for 0.2 µm sterile filtration.

### Example 14 (reference example)

The purpose of this example was to demonstrate the inability to sterile filter a nanoparticulate beclomethasone composition stabilized with polyvinylpyrrolidone.

Beclomethasone dipropionate (4.5 g) was dispersed in an aqueous solution of polyvinylpyrrolidone (0.90 g) in deionized water (84.6 g). The slurry was combined with 130 mL of 500 µm SDy20 polymeric media and charged into the 150 cc batch chamber of a DYNO®-Mill. Milling was performed at 4200 rpm. After 1 hour the dispersion had a mean particle size of 389 nm, and after two hours the mean particle size was 387 nm, indicating that no further size reduction had taken place. The dispersion was also highly aggregated.

Due to the large particle size and extent of aggregation the nanoparticulate beclomethasone dispersion was deemed unsuitable for 0.2 µm sterile filtration.

### Example 15 (reference example)

The purpose of this example was to demonstrate the inability to sterile filter a nanoparticulate flunisolide composition stabilized with tyloxapol.

Flunisolide is an anti-inflammatory steroid having the chemical name 6a-fluoro-11β, 16α, 17, 21-tetrahydroxy-pregna-1, 4-diene-3, 20-dione cyclic-16,17-acetal with acetone. It is practically insoluble in water.

Flunisolide (8.5 g) was dispersed in an aqueous solution of tyloxapol (1.7 g) and sodium chloride (1.53 g) in deionized water (73.27 g). The slurry was combined with 130 mL of 500 µm SDy20 polymeric media and charged into the 150 cc batch chamber of a DYNO®-Mill. Milling was performed at 4200 rpm. After 1.5 hours the nanoparticulate flunisolide dispersion was harvested and had a mean particle size of 115 nm.

Approximately 42.5 g of this nanoparticulate flunisolide dispersion was diluted with 42.5 g of deionized water and then combined with 120 mL of 50 µm SDy20 polymeric media. The material was charged into the 150 cc batch chamber of a DYNO®-Mill and milled at 4200 rpm. After 2 hours of milling the nanoparticulate flunisolide dispersion was harvested and had a mean particle size of 99 nm.

In spite of the relatively small particle size of the nanoparticulate flunisolide dispersion, the material could not be filtered through Gelman Supor 0.45 µm or 0.8/0.2 µm polyethersulfone syringe filters.

### Example 16 (reference example)

The purpose of this example was to demonstrate the inability to sterile filter a nanoparticulate triamcinolone acetonide composition stabilized with tyloxapol.

Triamcinolone acetonide is a corticosteroid with the chemical designation 9-Fluoro-11b 16a, 17, 21-tetrahydroxypregna-1, 4-diene-3, 20-dione cyclic 16, 17-acetal with acetone (C₁₄H₃₁FO₆).

Triamcinolone acetonide (4.25 g) was dispersed in an aqueous solution of tyloxapol (0.85 g) in deionized water (79.90 g). The slurry was combined with 130 cc of 500 µm SDy20 polymeric media and charged into the 150 cc batch chamber of a DYNO®-Mill. Milling was performed at 4200 rpm. After 1 hour the dispersion had a mean primary particle size of 164 nm but was highly aggregated, and after two hours the mean primary particle size was 157 nm, indicating that no significant additional size reduction had taken place.

The nanoparticulate triamcinolone acetonide dispersion remained highly aggregated with the average aggregate size being approximately 3 µm. Due to the large particle size and extent of aggregation the nanoparticulate triamcinolone acetonide dispersion was deemed unsuitable for 0.2 µm sterile filtration.

### Example 17 (reference example)

The purpose of this example was to demonstrate the inability to sterile filter a nanoparticulate triamcinolone acetonide composition stabilized with tyloxapol.

Triamcinolone acetonide (4.25 g) was dispersed in an aqueous solution of tyloxapol (2.13 g) in deionized water (78.62 g). The slurry was combined with 130 cc of 500 µm SDy20 polymeric media and charged into the 150 cc batch chamber of a DYNO®-Mill. Milling was performed at 4200 rpm. After 1 hour the dispersion had a mean primary particle size of 171 nm but was highly aggregated, and after two hours the mean primary particle size was 144 nm, indicating that very little additional size reduction had taken place.

The nanoparticulate triamcinolone acetonide dispersion remained highly aggregated with the average aggregate size being approximately 3.7 µm. Due to the large particle size and extent of aggregation the nanoparticulate triamcinolone acetonide dispersion was deemed unsuitable for 0.2 µm sterile filtration.

### Example 18 (reference example)

The purpose of this example was to demonstrate the inability to sterile filter a nanoparticulate triamcinolone acetonide composition stabilized with tyloxapol and the secondary surface stabilizer polyvinylpyrrolidone.

Triamcinolone acetonide (4.25 g) was dispersed in an aqueous solution of tyloxapol (0.85 g) and poylvinylpyrrolidone (0.85 g) in deionized water (79.05 g). The slurry was combined with 130 cc of 500 µm SDy20 polymeric media and charged into the 150 cc batch chamber of a DYNO®-Mill. Milling was performed at 4200 rpm. After 1 hour the dispersion had a mean primary particle size of 152 nm but was highly aggregated, and after two hours the mean primary particle size was 117 nm, indicating that relatively little additional size reduction had taken place.

The nanoparticulate triamcinolone acetonide dispersion remained highly aggregated with the average aggregate size being approximately 1 µm. Due to the large particle size and extent of aggregation the nanoparticulate triamcinolone acetonide dispersion was deemed unsuitable for 0.2 µm sterile filtration.

## Claims

1. A nanoparticulate composition comprising:
(a) nanoparticulate budesonide particles, having an effective average particle size of less than 150 nm; and
(b) tyloxapol as a surface stabilizer adsorbed onto the surface of said budesonide particles,
wherein the nanoparticulate composition is sterile filtered.

2. The composition of claim 1, wherein
(i) the budesonide particles are present in an amount selected from the group consisting of 99% to 1% (w/w), 90% to 10% (w/w), 80% to 30%, and 80% to 40% (w/w), based on the total combined dry weight of budesonide and tyloxapol; or
(ii) the concentration of tyloxapol is selected from the group consisting of from 0.01 to 90%, from 1 to 75%, from 10 to 60%, and from 10 to 30% by weight, based on the total combined dry weight of budesonide and tyloxapol; or
(iii) the budesonide particles are crystalline, semi-crystalline, amorphous, semi-amorphous, or a mixture thereof; or
(iv) the composition comprises one or more pharmaceutically acceptable excipients.

3. The composition of claim 1, wherein the effective average particle size of the budesonide particles is less than 120 nm, preferably less than 100 nm, more preferably less than 80 nm and most preferably less than 50 nm.

4. The composition of claim 1 further comprising at least one secondary surface stabilizer, preferably wherein the secondary surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylcellulose, hydroxypropylmethylcellulose phthalate, noncrystalline cellulose, magnesium aluminum silicate, triethanolamine, polyvinyl alcohol, polyvinylpyrrolidone, poloxamers, poloxamines, charged phospholipids, dioctylsulfosuccinate, Tetronic 1508(R), dialkylesters of sodium sulfosuccinic acid, sodium lauryl sulfates, alkyl aryl polyether sulfonates, mixtures of sucrose stearate and sucrose distearate, p-isononylphenoxypoly-(glycidol), C₁₈H₃₇CH₂(CON(CH₃)-CH₂(CHOH)₄(CH₂OH)₂, decanoyl-N-methylglucamide, n-decyl β-D-glucopyranoside, n-decyl β-D-maltopyranoside, n-dodecyl β-D-glucopyranoside, n-dodecyl β -D-maltoside, heptanoyl-N-methylglucamide, n-heptyl- β -D-glucopyranoside, n-heptyl β-D-thioglucoside, n-hexyl β-D-glucopyranoside, nonanoyl-N-methylglucamide, n-noyl β-D-glucopyranoside, octanoyl-N-methylglucamide, n-octyl- β-D-glucopyranoside, octyl β-D-thioglucopyranoside, and random copolymers of vinyl acetate and vinyl pyrrolidone, or selected from the group consisting of dioctylsulfosuccinate, sodium lauryl sulfate, hydroxypropylmethyl cellulose, benzalkonium chloride, and polyvinylpyrrolidine.

5. The composition of claim 1 formulated into an aerosol for nasal or pulmonary administration.

6. A method of making a nanoparticulate composition comprising:
(a) dispersing particles of budesonide in a liquid dispersion medium; and
(b) applying mechanical means in the presence of grinding media to reduce the average particle size of budesonide in the liquid dispersion medium to less than 150 nm, and
(c) sterile filtering the resulting nanoparticulate dispersion;
wherein tyloxapol is added to the liquid dispersion medium before or after milling.

7. A method of making a nanoparticulate composition comprising:
(a) dissolving budesonide in a solvent;
(b) adding the solubilized budesonide to a solution comprising tyloxapol to form a clear solution;
(c) precipitating the solubilized budesonide having tyloxapol adsorbed on the surface thereof using a non-solvent; and
(d) sterile filtering the resulting nanoparticulate dispersion,
wherein the resulting composition of nanoparticulate budesonide has an effective average particle size of less than 150 nm.

8. The method of claim 6 or 7, wherein
(i) the budesonide particles are present in an amount selected from the group consisting of 99% to 1% (w/w), 90% to 10% (w/w), 80% to 30%, and 80% to 40% (w/w), based on the total combined dry weight of budesonide and tyloxapol; or
(ii) the concentration of tyloxapol is selected from the group consisting of from 0.01 to 90%, from 1 to 75%, from 10 to 60%, and from 10 to 30% by weight, based on the total combined dry weight of budesonide and tyloxapol; or
(iii) the effective average particle size of the budesonide particles is selected from the group consisting of less than 120 nm, less than 100 nm, less than 80 nm, and less than 50 nm; or
(iv) the budesonide particles are crystalline, semi-crystalline, amorphous, semi-amorphous, or a mixture thereof.

9. The method of claim 6 or 7 further comprising adding at least one secondary surface stabilizer to the liquid dispersion medium before or after milling, preferably wherein the secondary surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hydroxypropyl methylcellulose, carboxymethylcellulose sodium, methylcellulose, hydroxyethylcellulose, hydroxypropylcellulose, hydroxypropylmethylcellulose phthalate, noncrystalline cellulose, magnesium aluminum silicate, triethanolamine, polyvinyl alcohol, polyvinylpyrrolidone, poloxamers, poloxamines, charged phospholipids, dioctylsulfosuccinate, Tetronic 1508(R), dialkylesters of sodium sulfosuccinic acid, sodium lauryl sulfates, alkyl aryl polyether sulfonates, mixtures of sucrose stearate and sucrose distearate, p-isononylphenoxypoly-(glycidol), C₁₈H₃₇CH₂(CON(CH₃)-CH₂(CHOH)₄(CH₂OH)₂, decanoyl-N-methylglucamide, n-decyl β-D-glucopyranoside, n-decyl β-D-maltopyranoside, n-dodecyl β-D-glucopyranoside, n-dodecyl β-D-maltoside, heptanoyl-N-methylglucamide, n-heptyl- β -D-glucopyranoside, n-heptyl β-D-thioglucoside, n-hexyl β -D-glucopyranoside, nonanoyl-N-methylglucamide, n-noyl β-D-glucopyranoside, octanoyl-N-methylglucamide, n-octyl- β-D-glucopyranoside, octyl β-D-thioglucopyranoside, and random copolymers of vinyl acetate and vinyl pyrrolidone or selected from the group consisting of dioctylsulfosuccinate, sodium lauryl sulfate, hydroxypropylmethyl cellulose, benzalkonium chloride, and polyvinylpyrrolidine.

10. A nanoparticulate composition for use in a method of treating a patient in need comprising administering to a patient in need a therapeutically effective amount of a nanoparticulate composition of budesonide, wherein said composition comprises:
(a) budesonide having an effective average particle size of less than 150 nm; and
(b) tyloxapol adsorbed on the surface of the budesonide,
wherein the nanoparticulate composition has been sterile filtered, preferably wherein said treatment is for an inflammatory disease, asthma, cystic fibrosis, or chronic obstructive pulmonary disease; or wherein said composition is administered via a nasal or pulmonary aerosol.

## Patentansprüche

1. Nanopartikuläre Zusammensetzung, umfassend:
(a) nanopartikuläre Budesonidpartikel mit einer effektiven durchschnittlichen Partikelgröße von weniger als 150 nm; und
(b) Tyloxapol als einen Oberflächenstabilisator, adsorbiert auf die Oberfläche der Budesonidpartikel,
wobei die nanopartikuläre Zusammensetzung sterilfiltriert ist.

2. Zusammensetzung nach Anspruch 1, wobei
(i) die Budesonidpartikel in einer Menge vorhanden sind, ausgewählt aus der Gruppe bestehend aus 99 % bis 1 % (Gew/Gew), 90 % bis 10 % (Gew/Gew), 80 % bis 30 % und 80 % bis 40 % (Gew/Gew), basierend auf dem gesamten kombinierten Trockengewicht von Budesonid und Tyloxapol; oder
(ii) die Konzentration von Tyloxapol ausgewählt ist aus der Gruppe bestehend aus von 0,01 bis 90 Gew.-%, von 1 bis 75 Gew.-%, von 10 bis 60 Gew.-% und von 10 bis 30 Gew.-%, basierend auf dem gesamten kombinierten Trockengewicht von Budesonid und Tyloxapol; oder
(iii) die Budesonidpartikel kristallin, semikristallin, amorph, semiamorph oder eine Mischung davon sind; oder
(iv) die Zusammensetzung einen oder mehrere pharmazeutisch verträgliche Hilfsstoffe umfasst.

3. Zusammensetzung nach Anspruch 1, wobei die effektive durchschnittliche Partikelgröße der Budesonidpartikel kleiner als 120 nm ist, vorzugsweise kleiner als 100 nm, stärker bevorzugt kleiner als 80 nm, am stärksten bevorzugt kleiner als 50 nm.

4. Zusammensetzung nach Anspruch 1, des Weiteren umfassend mindestens einen sekundären Oberflächenstabilisator, vorzugsweise wobei der sekundäre Oberflächenstabilisator ausgewählt ist aus der Gruppe bestehend aus Cetylpyridiniumchlorid, Gelatine, Kasein, Phosphatiden, Dextran, Glycerin, Akaziengummi, Cholesterin, Tragacanth, Stearinsäure, Benzalkoniumchlorid, Calciumstearat, Glycerinmonostearat, Cetostearylalkohol, Cetomacrogol emulgierendem Wachs, Sorbitanestern, Polyoxyethylenalkylethem, Polyoxyethylenkastorölderivativen, Polyoxyethylensorbitanfettsäureestern, Polyethylenglykolen, Dodecyltrimethylammoniumbromid, Polyoxyethylenstearaten, kolloidalem Siliciumdioxid, Phosphaten, Natriumdodecylsulfat, Carboxymethylcellulose Calcium, Hydroxypropylcellulosen, Hydroxypropylmethylcellulose, Carboxymethylcellulose Natrium, Methylcellulose, Hydroxyethylcellulose, Hydroxypropylcellulose, Hydroxypropylmethylcellulosephthalat, nichtkristalliner Cellulose, Magnesiumaluminiumsilicat, Triethanolamin, Polyvinylalkohol, Polyvinylpyrrolidon, Poloxameren, Poloxaminen, geladenen Phospholipiden, Dioctylsulfosuccinat, Tetronic 1508(R), Dialkylestern von Natriumsulfobernsteinsäure, Natriumlaurylsulfaten, Alkylarylpolyethersulfonaten, Mischungen von Sacharosestearat und Sacharosedistearat, p-Isononylphenoxypoly-(glycidol), C₁₈H₃₇CH₂(CON(CH₃)-CH₂(CHOH)₄(CH₂OH)₂, Decanoyl-N-methylglucamid, n-Decyl-β-D-glucopyranosid, n-Decyl-β-D-maltopyranosid, n-Dodecyl-β-D-glucopyranosid, n-Dodecyl-β-D-maltosid; Heptanoyl-N-methylglucamid, n-Heptyl-β-D-glucopyranosid, n-Heptyl-β-D-thioglucosid, n-Hexyl-β-D-glucopyranosid, Nonanoyl-N-methylglucamid; n-Noyl-β-D-glucopyranosid, Octanoyl-N-methylglucamid, n-Octyl-β-D-glucopyranosid, Octyl-β-D-thioglucopyranosid und Zufallscopolymeren von Vinylacetat und Vinylpyrrolidon, oder ausgewählt aus der Gruppe bestehend aus Dioctylsulfosuccinat, Natriumlaurylsulfat, Hydroxypropylmethylcellulose, Benzalkoniumchlorid und Polyvinylpyrrolidin.

5. Zusammensetzung nach Anspruch 1, formuliert in ein Aerosol zur nasalen oder pulmonalen Verabreichung.

6. Verfahren zum Herstellen einer nanopartikulären Zusammensetzung, umfassend:
(a) Dispergieren von Budesonidpartikeln in einem flüssigen Dispersionsmedium; und
(b) Anwenden von mechanischen Mitteln in der Gegenwart von Mahlmedien zum Reduzieren der durchschnittlichen Partikelgröße von Budesonid in dem flüssigen Dispersionsmedium auf weniger als 150 nm; und
(c) Sterilfiltrieren der resultierenden nanopartikulären Dispersion,
wobei Tyloxapol zu dem flüssigen Dispersionsmedium vor oder nach dem Mahlen hinzugefügt wird.

7. Verfahren zum Erzeugen einer nanopartikulären Zusammensetzung, umfassend:
(a) Lösen von Budesonid in einem Lösungsmittel;
(b) Hinzufügen des gelösten Budesonids zu einer Lösung, umfassend Tyloxapol, unter Bildung einer klaren Lösung;
(c) Präzipitieren des gelösten Budesonids mit auf der Oberfläche davon adsorbiertem Tyloxapol unter Verwendung eines Nicht-Lösungsmittels; und
(d) Sterilfiltrieren der resultierenden nanopartikulären Dispersion,
wobei die resultierende Zusammensetzung von nanopartikulärem Budesonid eine effektive durchschnittliche Partikelgröße von weniger als 150 nm aufweist.

8. Verfahren nach Anspruch 6 oder 7, wobei
(i) die Budesonidpartikel in einer Menge vorhanden sind, ausgewählt aus der Gruppe bestehend aus 99 % bis 1 % (Gew/Gew), 90 % bis 10 % (Gew/Gew), 80 % bis 30 % und 80 % bis 40 % (Gew/Gew), basierend auf dem gesamten kombinierten Trockengewicht von Budesonid und Tyloxapol; oder
(ii) die Konzentration von Tyloxapol ausgewählt ist aus der Gruppe bestehend aus von 0,01 bis 90 Gew.-%, von 1 bis 75 Gew.-%, von 10 bis 60 Gew.-% und von 10 bis 30 Gew.-%, basierend auf dem gesamten kombinierten Trockengewicht von Budesonid und Tyloxapol; oder
(iii) die effektive durchschnittliche Partikelgröße der Budesonidpartikel ausgewählt ist aus der Gruppe bestehend aus kleiner als 120 nm, kleiner als 100 nm, kleiner als 80 nm und kleiner als 50 nm; oder
(iv) die Budesonidpartikel kristallin, semikristallin, amorph, semiamorph oder eine Mischung davon sind.

9. Verfahren nach Anspruch 6 oder 7, des Weiteren umfassend das Hinzufügen mindestens eines sekundären Oberflächenstabilisators zu dem flüssigen Dispersionsmedium vor oder nach dem Mahlen, vorzugsweise wobei der sekundäre Oberflächenstabilisator ausgewählt ist aus der Gruppe bestehend aus Cetylpyridiniumchlorid, Gelatine, Kasein, Phosphatiden, Dextran, Glycerin, Akaziengummi, Cholesterin, Tragacanth, Stearinsäure, Benzalkoniumchlorid, Calciumstearat, Glycerinmonostearat, Cetostearylalkohol, Cetomacrogol emulgierendem Wachs, Sorbitanester, Polyoxyethylenalkylethem, Polyoxyethylenkastorölderivativen, Polyoxyethylensorbitanfettsäureestern, Polyethylenglykolen, Dodecyltrimethylammoniumbromid, Polyoxyethylenstearaten, kolloidalem Siliciumdioxid, Phosphaten, Natriumdodecylsulfat, Carboxymethylcellulose Calcium, Hydroxypropylcellulosen, Hydroxypropylmethylcellulose, Carboxymethylcellulose Natrium, Methylcellulose, Hydroxyethylcellulose, Hydroxypropylcellulose, Hydroxypropylmethylcellulosephthalat, nichtkristalliner Cellulose, Magnesiumaluminiumsilicat, Triethanolamin, Polyvinylalkohol, Polyvinylpyrrolidon, Poloxameren, Poloxaminen, geladenen Phospholipiden, Dioctylsulfosuccinat, Tetronic 1508(R), Dialkylestern von Natriumsulfobernsteinsäure, Natriumlaurylsulfaten, Alkylarylpolyethersulfonaten, Mischungen von Sacharosestearat und Sacharosedistearat, p-Isononylphenoxypoly-(glycidol), C₁₈H₃₇CH₂(CON(CH₃)-CH₂(CHOH)₄(CH₂OH)₂, Decanoyl-N-methylglucamid, n-Decyl-β-D-glucopyranosid, n-Decyl-β-D-maltopyranosid, n-Dodecyl-β-D-glucopyranosid, n-Dodecyl-β-D-maltosid; Heptanoyl-N-methylglucamid, n-Heptyl-β-D-glucopyranosid, n-Heptyl-β-D-thioglucosid, n-Hexyl-β-D-glucopyranosid, Nonanoyl-N-methylglucamid; n-Noyl-β-D-glucopyranosid, Octanoyl-N-methylglucamid, n-Octyl-β-D-glucopyranosid, Octyl-β-D-thioglucopyranosid und Zufallscopolymeren von Vinylacetat und Vinylpyrrolidon, oder ausgewählt aus der Gruppe bestehend aus Dioctylsulfosuccinat, Natriumlaurylsulfat, Hydroxypropylmethylcellulose, Benzalkoniumchlorid und Polyvinylpyrrolidin.

10. Nanopartikuläre Zusammensetzung zur Verwendung in einem Verfahren zum Behandeln eines bedürftigen Patienten, umfassend das Verabreichen einer therapeutisch wirksamen Menge einer nanopartikulären Zusammensetzung von Budesonid an einen bedürftigen Patienten, wobei die Zusammensetzung umfasst:
(a) Budesonid mit einer effektiven durchschnittlichen Partikelgröße von weniger als 150 nm; und
(b) Tylaxapol, adsorbiert an die Oberfläche des Budesonid,
wobei die nanopartikuläre Zusammensetzung sterilfiltriert worden ist, vorzugsweise
wobei die Behandlung für eine inflammatorische Erkrankung, Asthma, zystische Fibrose oder chronisch obstruktive pulmonale Erkrankung ist; oder wobei die Zusammensetzung mittels eines nasalen oder pulmonalen Aerosols verabreicht wird.

## Revendications

1. Composition nanoparticulaire comprenant :
(a) des particules nanoparticulaires de budésonide, ayant une taille de particules moyenne effective inférieure à 150 nm ; et
(b) du tyloxapol comme stabilisant de surface adsorbé sur la surface desdites particules de budésonide,
dans laquelle la composition nanoparticulaire est stérilisée par filtration.

2. Composition selon la revendication 1, dans laquelle
(i) les particules de budésonide sont présentes dans une quantité choisie dans le groupe constitué de 99 % à 1 % (m/m), 90 % à 10 % (m/m), 80 % à 30 %, et 80 % à 40 % (m/m), sur la base du poids sec combiné total de budésonide et de tyloxapol ; ou
(ii) la concentration de tyloxapol est choisie dans le groupe constitué de 0, 01 à 90 %, de 1 à 75 %, de 10 à 60 %, et de 10 à 30 % en poids, sur la base du poids sec combiné total de budésonide et de tyloxapol ; ou
(iii) les particules de budésonide sont cristallines, semi-cristallines, amorphes, semi-amorphes, ou un mélange de celles-ci ; ou
(iv) la composition comprend un ou plusieurs excipients pharmaceutiquement acceptables.

3. Composition selon la revendication 1, dans laquelle la taille de particules moyenne effective des particules de budésonide est inférieure à 120 nm, de préférence inférieure à 100 nm, plus préférablement inférieure à 80 nm et de manière préférée entre toutes inférieure à 50 nm.

4. Composition selon la revendication 1, comprenant en plus au moins un stabilisant de surface secondaire, où de préférence le stabilisant de surface secondaire est choisi dans le groupe constitué par le chlorure de cétylpyridinium, la gélatine, la caséine, les phosphatides, le dextran, le glycérol, la gomme arabique, le cholestérol, l'adragante, l'acide stéarique, le chlorure de benzalkonium, le stéarate de calcium, le monostéarate de glycérol, l'alcool cétostéarylique, une cire émulsionnante au cétomacrogol, les esters de sorbitan, les alkyléthers polyoxyéthylénés, les dérivés d'huile de ricin polyoxyéthylénés, les esters d'acide gras et de sorbitan polyoxyéthylénés, les polyéthylèneglycols, le bromure de dodécyltriméthylammonium, les stéarates polyoxyéthylénés, le dioxyde de silicium colloïdal, les phosphates, le dodécylsulfate de sodium, la carboxyméthylcellulose calcique, les hydroxypropylcelluloses, l'hydroxylpropylméthylcellulose, la carboxyméthylcellulose sodique, la méthylcellulose, l'hydroxyéthylcellulose, l'hydroxypropylcellulose, le phtalate d'hydroxypropylméthylcellulose, la cellulose non cristalline, le silicate d'aluminium et de magnésium, la triéthanolamine, le poly(alcool de vinyle), la polyvinylpyrrolidone, les poloxamères, les poloxamines, les phospholipides chargés, un dioctylsulfosuccinate, le Tetronic 1508(R), les dialkylesters de l'acide sulfosuccinique sodique, les laurylsulfates de sodium, les alkyl aryl polyéther sulfonates, les mélanges de stéarate de saccharose et distéarate de saccharose, un p-isononylphénoxypoly-(glycidol), C₁₈H₃₇CH₂ (CON (CH₃) - CH₂(CHOH)₄(CH₂OH)₂, le décanoyl-N-méthylglucamide, le n-décyle β-D-glucopyranoside, le n-décyle β-D-maltopyranoside, le n-dodécyle β-D-glucopyranoside, le n-dodécyle β-D-maltoside, l'heptanoyl-N-méthylglucamide, le n-heptyle β-D-glucopyranoside, le n-heptyle β-D-thioglucoside, le n-hexyle β-D-glucopyranoside, le nonanoyl-N-méthylglucamide, le n-noyle β-D-glucopyranoside, l'octanoyl-N-méthylglucamide, le n-octyle β-D-glucopyranoside, l'octyle β-D-thioglucopyranoside, et les copolymères aléatoires d'acétate de vinyle et de vinylpyrrolidone, ou choisi dans le groupe constitué d'un dioctylsulfosuccinate, du laurylsulfate de sodium, de l'hydroxypropylméthylcellulose, du chlorure de benzalkonium, et de la polyvinylpyrrolidine.

5. Composition selon la revendication 1 formulée en un aérosol pour une administration nasale ou pulmonaire.

6. Procédé de fabrication d'une composition nanoparticulaire comprenant :
(a) la dispersion de particules de budésonide dans un milieu de dispersion liquide ; et
(b) l'application d'un moyen mécanique en présence d'un milieu de broyage pour réduire la taille de particules moyenne du budésonide dans le milieu de dispersion liquide à moins de 150 nm, et
(c) la filtration stérilisante de la dispersion nanoparticulaire résultante ;
dans lequel du tyloxapol est ajouté au milieu de dispersion liquide avant ou après broyage.

7. Procédé de fabrication d'une composition nanoparticulaire comprenant :
(a) la dissolution de budésonide dans un solvant ;
(b) l'ajout du budésonide solubilisé à une solution comprenant du tyloxapol pour former une solution limpide ;
(c) la précipitation du budésonide solubilisé ayant du tyloxapol adsorbé sur la surface de celui-ci en utilisant un non solvant ; et
(d) la filtration stérilisante de la dispersion nanoparticulaire résultante,
dans lequel la composition résultante de budésonide nanoparticulaire a une taille de particules moyenne effective inférieure à 150 nm.

8. Procédé selon la revendication 6 ou 7, dans lequel
(i) les particules de budésonide sont présentes dans une quantité choisie dans le groupe constitué de 99 % à 1 % (m/m), 90 % à 10 % (m/m), 80 % à 30 %, et 80 % à 40 % (m/m), sur la base du poids sec combiné total de budésonide et de tyloxapol ; ou
(ii) la concentration de tyloxapol est choisie dans le groupe constitué de 0,01 à 90 %, de 1 à 75 %, de 10 à 60 %, et de 10 à 30 % en poids, sur la base du poids sec combiné total de budésonide et de tyloxapol ; ou
(iii) la taille de particules moyenne effective des particules de budésonide est choisie dans le groupe constitué de inférieure à 120 nm, inférieure à 100 nm, inférieure à 80 nm, et inférieure à 50 nm ; ou
(iv) les particules de budésonide sont cristallines, semi-cristallines, amorphes, semi-amorphes, ou un mélange de celles-ci.

9. Procédé selon la revendication 6 ou 7, comprenant en plus l'ajout d'au moins un stabilisant de surface secondaire au milieu de dispersion liquide avant ou après le broyage, où de préférence le stabilisant de surface secondaire est choisi dans le groupe constitué par le chlorure de cétylpyridinium, la gélatine, la caséine, les phosphatides, le dextran, le glycérol, la gomme arabique, le cholestérol, l'adragante, l'acide stéarique, le chlorure de benzalkonium, le stéarate de calcium, le monostéarate de glycérol, l'alcool cétostéarylique, une cire émulsionnante au cétomacrogol, les esters de sorbitan, les alkyléthers polyoxyéthylénés, les dérivés d'huile de ricin polyoxyéthylénés, les esters d'acide gras et de sorbitan polyoxyéthylénés, les polyéthylèneglycols, le bromure de dodécyltriméthylammonium, les stéarates polyoxyéthylénés, le dioxyde de silicium colloïdal, les phosphates, le dodécylsulfate de sodium, la carboxyméthylcellulose calcique, les hydroxypropylcelluloses, l'hydroxylpropylméthylcellulose, la carboxyméthylcellulose sodique, la méthylcellulose, l'hydroxyéthylcellulose, l'hydroxypropylcellulose, le phtalate d'hydroxypropylméthylcellulose, la cellulose non cristalline, le silicate d'aluminium et de magnésium, la triéthanolamine, le poly(alcool de vinyle), la polyvinylpyrrolidone, les poloxamères, les poloxamines, les phospholipides chargés, un dioctylsulfosuccinate, le Tetronic 1508(R), les dialkylesters de l'acide sulfosuccinique sodique, les laurylsulfates de sodium, les alkyl aryl polyéther sulfonates, les mélanges de stéarate de saccharose et distéarate de saccharose, un p-isononylphénoxypoly- (glycidol), C₁₈H₃₇CH₂ (CON (CH₃)-CH₂(CHOH)₄(CH₂OH)₂, le décanoyl-N-méthylglucamide, le n-décyle β-D-glucopyranoside, le n-décyle β-D-maltopyranoside, le n-dodécyle β-D-glucopyranoside, le n-dodécyle β-D-maltoside, l'heptanoyl-N-méthylglucamide, le n-heptyle β-D-glucopyranoside, le n-heptyle β-D-thioglucoside, le n-hexyle β-D-glucopyranoside, le nonanoyl-N-méthylglucamide, le n-noyle β-D-glucopyranoside, l'octanoyl-N-méthylglucamide, le n-octyle β-D-glucopyranoside, l'octyle β-D-thioglucopyranoside, et les copolymères aléatoires d'acétate de vinyle et de vinylpyrrolidone, ou choisi dans le groupe constitué d'un dioctylsulfosuccinate, du laurylsulfate de sodium, de l'hydroxypropylméthylcellulose, du chlorure de benzalkonium, et de la polyvinylpyrrolidine.

10. Composition nanoparticulaire pour une utilisation dans un procédé de traitement d'un patient en ayant besoin comprenant l'administration à un patient en ayant besoin, d'une quantité thérapeutiquement efficace d'une composition nanoparticulaire de budésonide, dans laquelle ladite composition comprend :
(a) du budésonide ayant une taille de particules moyenne effective inférieure à 150 nm ; et
(b) du tyloxapol adsorbé sur la surface du budésonide,
dans laquelle la composition nanoparticulaire a été stérilisée par filtration, de préférence dans laquelle ledit traitement est destiné à une maladie inflammatoire, un asthme, une fibrose kystique, ou une bronchopneumopathie chronique obstructive ; ou dans laquelle ladite composition est administrée par l'intermédiaire d'un aérosol nasal ou pulmonaire.
